# EUROPEAN PATENT SPECIFICATION

(11) **EP 0 400 829 B1**
(45) Date of publication and mention of the grant of the patent: **02.03.1994**
(21) Application number: 90305093.8
(22) Date of filing: 11.05.1990
(51) Int. Cl.: C12M 1/38

(54) **Temperature maintaining method for bioreactor**
Verfahren zur Konstanthaltung der Temperatur in einem Bioreaktor
Procédé pour maintenir la température dans un bioréacteur

(30) Priority: 12.05.1989 JP 119950/89
(43) Date of publication of application: 05.12.1990
(73) Proprietor: NGK INSULATORS, LTD., Nagoya City Aichi Pref. (JP)
(72) Inventor: Matsuoka, Keiji, Kani City, Gifu Pref. (JP); Kawase, Mitsuo, Chita City, Aichi Pref. (JP); Niwa, Isao, Tajimi City, Gifu Pref. (JP)
(74) Representative: Paget, Hugh Charles Edward

(56) References cited:
- FR-A- 977 574
- FR-A- 1 003 620
- FR-A- 1 004 411
- FR-A- 1 016 039
- FR-A- 1 172 210
- FR-A- 2 209 835
- FR-A- 2 376 209

## Description

This invention relates to a method of maintaining a liquid at a constant temperature in a bioreactor, particularly operated under anaerobic conditions.

In the case of a bioreactor for anaerobic treatment, for example using high temperature methane bacteria, it is desirable to maintain the temperature in the reactor within a fairly close tolerance. In practice, the temperature is controlled in a range such as 55°C ± 2°. In order to maintain the temperature in the bioreactor within a constant temperature zone, however, heating or cooling is needed for various reasons, such as a change in temperature of raw water. It is difficult to automatically change over heating and cooling to control the temperature in the bioreactor exactly.

FR-A- 1 172 210 discloses a method for periodically varying the temperature of a mould over a relatively large temperature difference.

It is an object of the invention to provide a method of maintaining a liquid in a bioreactor at a constant temperature, which ameliorates the disadvantages of the prior art and is able to keep liquid temperature in the reactor within a constant temperature zone with close tolerance with a relatively simple means.

The method of the present invention is set out in claim 1.

The invention will be more fully understood by referring to the following detailed specification and claims taken in connection with the appended drawings.
Fig. 1 is a drawing of a control system illustrating one embodiment of the invention; and
Fig. 2 is a graph illustrating the hot and cold water temperature control systems switching on and off in response to change in temperature of a liquid in a bioreactor.

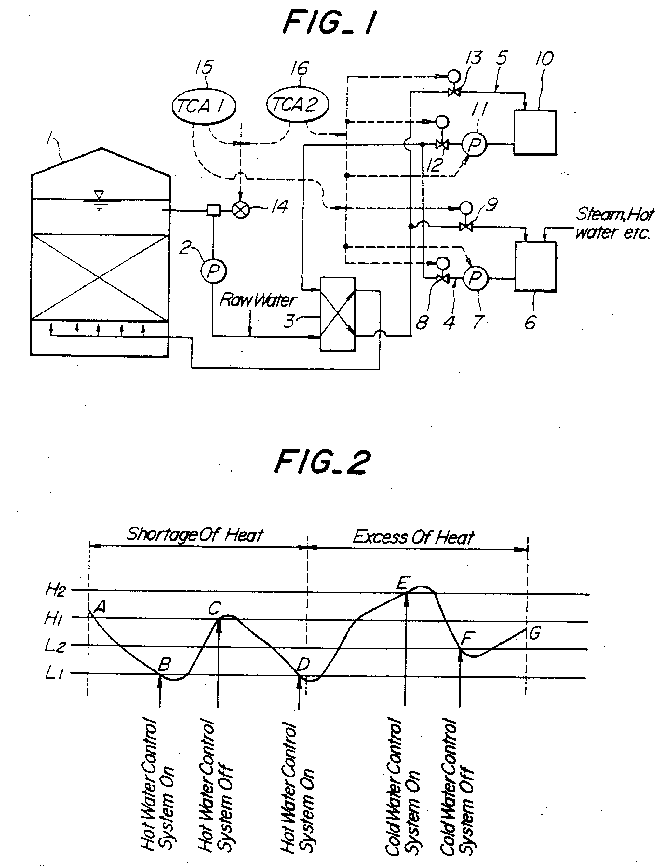

Fig. 1 illustrates an apparatus for carrying out the method according to the invention, which comprises a bioreactor 1 for anaerobic treatment, a pump 2 for circulating a liquid in the bioreactor and a heat exchanger 3. The liquid in the bioreactor 1 is fed by the pump 2 into the heat exchanger 3 and cooled or heated therein and the liquid is returned into the bottom of the bioreactor. The heat exchanger 3 is connected to a hot water temperature control system 4 and a cold water temperature control system 5.

The hot water temperature control system 4 comprises a hot water tank 6, a hot water pump 7, a hot water valve 8, a hot water return valve 9 and a controller 15. On the other hand, the cold water temperature control system 5 comprises a cooling tower 10, a cold water pump 11, a cold water valve 12, a cold water return valve 13 and a controller 16.
The controllers 15 and 16 for the systems 4 and 5 are actuated in response to signals emitted from a thermometer 14 of the bioreactor 1 to turn the pump and the valves on and off.

It is assumed that the hot water temperature control system 4 becomes on at a temperature L₁ and off at a temperature H₁, while the cold water temperature control system 5 becomes off at a temperature L₂ and on at a temperature H₂. According to the invention, these temperatures are preferably set in an order of L₁, L₂, H₁ and H₂ from low to high temperatures. They are practically set, for example L₁=53°C, L₂=54°C, H₁=55°C and H₂=56°C.

The operation of the apparatus shown in Fig. 1 will then be explained with reference to Fig. 2.

First, when the liquid temperature under insufficient quality of heat lowers progressively from a point A to point B or the temperature L₁, the hot water temperature control system will turn on. As a result, the hot water valve 8 and the hot water return valve 9 are opened and the hot water pump 7 is actuated to feed hot water to the heat exchanger 3 so that the liquid temperature in the bioreactor starts to rise progressively. When the liquid temperature has attained H₁ at C in this manner, the hot water temperature control system turns off.

Second, when the liquid temperature under excessive quality of heat attends H₂ at E, the cold water temperature control system turns on. As a result, the cold water valve 12 and the cold water return valve 13 are opened and the cold water pump 11 is actuated to feed cold water into the heat exchanger 3 so that the liquid temperature in the bioreactor lowers progressively. When the liquid temperature has attained L₂ at F in this manner, the cold water temperature control system turns off. The liquid temperature start to rise again.

As above described, according to the invention the liquid temperature in the bioreactor can be maintained in the range between L₁ and H₂ by only turning the hot and cold water temperature control systems on and off. Even if excess or shortage of heat occurs, exact temperature control suitable for activity of anaerobic bacteria may be performed with ease.

By using the cooling tower 10 as in the embodiment above described, consumption of the cooling water may be substantially prevented. Moreover, by utilizing combustible gases produced in the bioreactor 1, the apparatus for carrying out the invention may need no external energy source.

As can be seen from the above explanation, the method according to the invention can maintain the liquid temperature of the bioreactor, whose temperature control is generally very important, within a constant temperature zone in a relatively simple manner. Moreover, even if excess or shortage of quality of heat in a bioreactor takes places, it may be overcome by the control according to the invention. Therefore, the invention greatly contributes to improvement of industries as a bioreactor temperature maintaining method which ameliorates the disadvantages of the prior art.

While the invention has been particularly shown and described with reference to preferred embodiments thereof, it will be understood by those skilled in the art that the foregoing and other changes in form and details can be made.

## Claims

1. A method of maintaining a liquid in a bioreactor within a constant temperature zone, comprising the steps of connecting a hot water temperature control system and a cold water temperature control system through a heat exchanger to a circulating line for the liquid in the bioreactor, setting a control temperature L₁ at which the hot water temperature control system becomes on, a control temperature L₂ at which the cold water temperature control system becomes off, a control temperature H₁ at which the hot water temperature control system becomes off, and a control temperature H₂ at which the cold water temperature control system becomes on, and turning the hot and cold water temperature control systems on and off when the temperature of the liquid in the bioreactor reaches these control temperatures and wherein said control temperatures are set in the order L₁, L₂, H₁ and H₂ or the order L₁, H₁, L₂ and H₂ from low to high temperature.

2. A method as set forth in claim 1, wherein said hot water temperature control system comprises a hot water tank, a hot water pump, a hot water valve, a hot water return valve and a controller.

3. A method as set forth in claim 1, wherein said cold water temperature control system comprises a cooling tower, a cold water pump, a cold water valve, a cold water return valve and a controller.

4. A method as set forth in claim 2 or 3, wherein said controller is actuated in response to signals emitted from a thermometer provided for measuring temperature of the liquid in the bioreactor to turn the pumps and the valves on and off.

## Patentansprüche

1. Verfahren, um eine Flüssigkeit in einem Bioreaktor in einem konstanten Temperaturbereich zu halten, folgende Schritte umfassend: das Verbinden eines Warmwassertemperaturregelsystems und eines Kaltwassertemperaturregelsystems über einen Wärmetauscher mit einer Umlaufleitung für die Flüssigkeit in einem Bioreaktor, das Festlegen einer Regeltemperatur L₁, bei der das Warmwassertemperaturregelsystem eingeschaltet wird, einer Regeltemperatur L₂, bei der das Kaltwassertemperaturregelsystem ausgeschaltet wird, einer Regeltemperatur H₁, bei das Warmwassertemperaturregelsystem ausgeschaltet wird und einer Regeltemperatur H₂, bei der das Kaltwassertemperaturregelsystem eingeschaltet wird, und das Ein- und Ausschalten des Warm- und des Kaltwassertemperaturregelsystems, wenn die Temperatur der Flüssigkeit im Bioreaktor diese Regeltemperaturen erreicht, und worin die genannten Regeltemperaturen von niedriger zu hoher Temperatur in der Reihenfolge L₁, L₂, H₁ und H₂ oder der Reihenfolge L₁, H₁, L₂ und H₂ festgelegt sind.

2. Verfahren nach Anspruch 1, worin das genannt Warmwassertemperaturregelsystem einen Warmwasserbehälter, eine Warmwasserpumpe, ein Warmwasserventil, ein Warmwasserrücklaufventil und einen Regler umfaßt.

3. Verfahren nach Anspruch 1, worin das genannte Kaltwassertemperaturregelsystem einen Kühlturm, eine Kaltwasserpumpe, ein Kaltwasserventil, ein Kaltwasserrücklaufventil und einen Regler umfaßt.

4. Verfahren nach Anspruch 2 oder 3, worin der genannte Regler als Reaktion auf Signale betätigt wird, die von einem Thermometer ausgesandt werden, das zum Messen der Temperatur der Flüssigkeitkeit im Bioreaktor vorgesehen ist, um die Pumpen und die Ventil ein- und auszuschalten.

## Revendications

1. Procédé de maintien d'un liquide dans un bioréacteur dans une zone de température constante, comprenant les étapes de relier un système de contrôle de température d'eau chaude et un système de contrôle de température d'eau froide, par à un échangeur de chaleur, à une ligne de circulation du liquide dans le bioréacteur, d'établir une température de contrôle L₁ à laquelle le système de contrôle de température d'eau chaude est activé, une température de contrôle L₂ à laquelle le système de contrôle de température d'eau froide est activé, une température de contrôle H₁ à laquelle le système de contrôle de température d'eau chaude est désactivé, et une température de contrôle H₂ à laquelle le système de contrôle de température d'eau froide est activé, et d'activer ou désactiver les systèmes de température d'eau froide et chaude lorsque la température du liquide dans le bioréacteur atteint ces températures de contrôle et dans lequel lesdites températures de contrôle sont établies dans l'ordre L₁, L₂ , H₁ , H₂ ou dans l'ordre L₁, H₁, L₂, H₂ en partant des basses températures vers les hautes températures.

2. Procédé selon la revendication 1, dans lequel ledit système de contrôle de température d'eau chaude comprend un réservoir d'eau chaude, une pompe d'eau chaude, une vanne d'eau chaude, une vanne de retour d'eau chaude et un contrôleur.

3. Procédé selon la revendication 1, dans lequel ledit système de contrôle de température d'eau froide comprend une tour de refroidissement, une pompe d'eau froide , une vanne d'eau froide , une vanne de retour d'eau froide et un contrôleur.

4. Procédé selon la revendication 2 ou 3, dans lequel ledit contrôleur est actionné en réponse à des signaux émis par un thermomètre disposé pour mesurer la température du liquide dans le bioréacteur pour activer et désactiver les pompes et les vannes.
